# EUROPEAN PATENT SPECIFICATION

(11) **EP 3 075 822 B1**
(45) Date of publication and mention of the grant of the patent: **27.02.2019**
(21) Application number: 14865908.9
(22) Date of filing: 29.09.2014
(51) Int. Cl.: C07C 31/125, C11B 9/00, C07C 29/17, C07C 45/74, C07C 49/203, C11D 3/00, C11D 3/50, D06M 13/00, D06M 13/144

(54) **PERFUME COMPOSITION**
DUFTSTOFFZUSAMMENSETZUNG
COMPOSITION DE PARFUM

(30) Priority: 29.11.2013 JP 2013248002
(43) Date of publication of application: 05.10.2016
(73) Proprietor: Kao Corporation, Chuo-Ku Tokyo 103-8210 (JP)
(72) Inventor: UEDA, Junko, Wakayama-shi, Wakayama 640-8580 (JP); ASADA, Takahiro, Wakayama-shi, Wakayama 640-8580 (JP); TOKI, Naotoshi, Cincinnati, OH 45214 (US); FUKUDA, Kazuyuki, Sumida-ku, Tokyo 131-8501 (JP)
(74) Representative: Hoffmann Eitle
(86) International application number: PCT/JP2014/075933
(87) International publication number: WO 2015/079798

(56) References cited:
- JP-A- H02 196 740
- JP-A- 2002 327 193
- JP-A- 2003 113 392
- JP-A- 2011 504 469
- JP-A- 2012 087 228
- JP-A- 2012 526 187
- JP-A- 2013 136 850
- US-A- 3 452 105

## Description

### Technical Field

The present invention relates to a fragrance composition containing 4,7-dimethyloctan-3-ol.

### Background Art

Fragrance is an important element that produces, for example, preference, a sense of luxury, a sense of ease, and expectations for the effect for products and the like. Furthermore, a distinctive fragrance provides an effect of differentiating products and the capacity for attracting customers. Particularly, floral fragrance notes are preferred for toiletry products.

Alcohols such as linalool, citronellol, and geraniol are known as fragrance materials that impart floral fragrance notes.

On the other hand, in order to control, for example, a long-lasting property and balance of a fragrance, generally, a fragrance is imparted to a product using a fragrance composition in which a plurality of fragrance materials are mixed together. It is required for the fragrance materials composing the fragrance composition to be highly harmonious with other fragrance materials.

Examples of commercial products of alcohols include 3,7-dimethyloctane-3-ol, which is a fragrance having a floral note and sold as tetrahydrolinalool. Further,
Patent Document 1 discloses that 2,5,7-trimethyl-3-octanol and 2,4-dimethyl-8-nonanol each are useful as a fragrance and have a fruit-like fragrance note.
Patent Document 2 discloses that, for example, 3-butyl-6-methylheptane-2-ol is useful as a fragrance and has green narcissus and aromatic gentian like fragrance notes.
Patent Document 3 discloses that 3-hydroxy-4-ethyl-7-methyloctane has a fresh flowery and fruit-like fragrance note similar to gooseberry, and secondarily has a herb-like odor.

Very roughly speaking, fragrance materials have similar fragrance notes when they have similar structures to each other, but there are many exceptions. Particularly, when a plurality of substituents are combined to change the structure, it is difficult to predict how the fragrance note will change and it is also difficult to predict the harmonicity with other fragrance materials.

### Prior Art Documents

### Patent Documents

Patent Document 1: JP 1979(S54)-27506 A
Patent Document 2: WO 2011/135487
Patent Document 3: U.S. Patent No. 3,452,105

### Disclosure of Invention

### Problem to be Solved by the Invention

Some fragrance materials may interfere with other fragrance components and thereby may change fragrance notes or weaken them mutually. Therefore, in order to increase flexibility in blending odors, it is desired to increase the number of types of fragrance materials. Particularly, sweet and bright flowery fragrance notes are very important as fragrances for toiletry products, and fresh citrus fragrance notes and accents of green leaves fragrance notes are also important. Fragrance materials having such floral fragrance notes and also having citrus and green leaves fragrance notes have been desired. On the other hand, fragrance compositions having fresh floral fragrance notes with a feeling of cleanliness and being suitably used to impart fragrances to toiletry products have been also desired.

Therefore, it is an object of the present invention to provide a fragrance composition that has citrus and green odors as well as a floral-like odor in good balance, has excellent harmonicity with various other fragrances, emphasizes odors by being blended, and can impart a fresh floral feeling.

### Means for Solving Problem

The present inventors found that saturated aliphatic alcohol with a specific structure has citrus and green odors as well as a floral-like odor in good balance, has excellent harmonicity with various other fragrances, emphasizes odors by being blended, and imparts a fresh floral feeling, and thereby the present invention was completed.

That is, it is an object of the present invention to provide a fragrance composition containing 4,7-dimethyloctan-3-ol.

### Effects of the Invention

The fragrance composition of the present invention contains 4,7-dimethyloctan-3-ol and therefore has excellent harmonicity with various other fragrances, emphasizes odors by being blended, and can impart a fresh floral feeling.

### Description of the Invention

### [Method of Producing 4,7-dimethyloctan-3-ol]

The 4,7-dimethyloctan-3-ol (represented by Formula (I) below. In the present description, it may also be referred to as Compound (I).) of the present invention can be synthesized using a common organic chemical reaction. The method of producing it is not limited. For example, Compound (I) can be produced using a method including a step of obtaining Compound (I) by carrying out a cross aldol reaction using isovaleraldehyde (represented by Formula (III) below. In the present description, it may also be referred to as Coumpound (III).) and 3-pentanone (represented by Formula (IV) below. In the present description, it may also be referred to as Compound (IV).), and then dehydrating and reducing it (see Scheme 1).

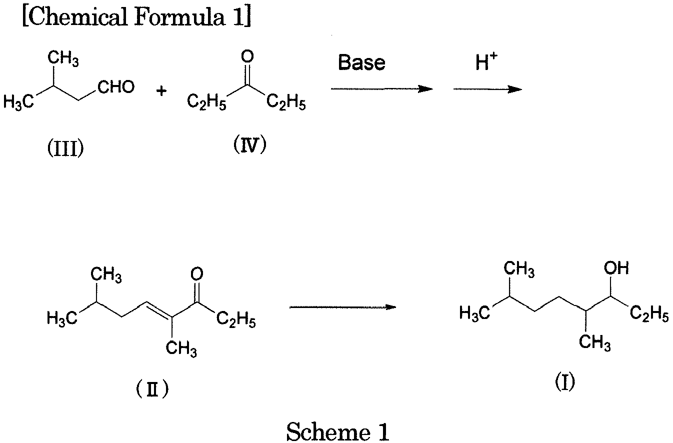

In the aforementioned production method, first, the cross aldol reaction is carried out using isovaleraldehyde (III) and 3-pentanone (IV) in the presence of a base catalyst. The base catalyst is preferably alkali metal hydroxide, and more preferably sodium hydroxide. The reaction is carried out at a temperature of, for example, 15 to 30°C for, for example, 1 to 60 hours.

Particularly, from the viewpoint of making the cross aldol reaction proceed with a good yield, it is preferable to further add water to the reaction solution.

Subsequently, the product thus obtained is dehydrated. Preferably, the dehydration is carried out at the temperature not lower than the boiling point of water, with, for example, phosphoric acid being added to the reaction solution. When the pressure of the reaction system is changed, it is preferable that the dehydration be carried out at the temperature not lower than the boiling point of water that is obtained under such pressure. When it is carried out at 1 atm (101 kPa), it is preferable that the product be dehydrated while being heated, for example, at 110 to 160°C.

The dehydration allows 4,7-methyloct-4-en-3-one (represented by Formula (II) above. In the present description, it may also be referred to as Compound (II).) to be obtained.

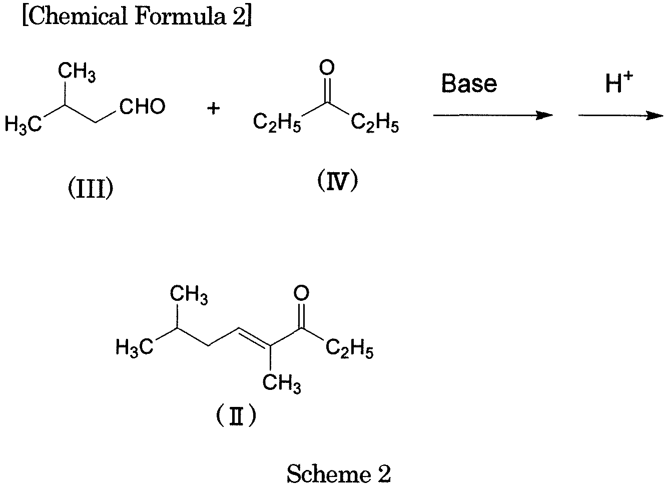

Subsequently, Compound (II) is reduced and thereby Compound (I) is obtained. The reduction method is not particularly limited as long as it is a general reduction method. However, from the viewpoint of increasing the purity, a hydrogenation method using a noble metal catalyst is preferred. Specifically, reduction can be carried out by hydrogenation under a hydrogen atmosphere in the presence of a noble metal catalyst such as ruthenium, palladium, or platinum. Hydrogenation is carried out under a hydrogen pressure of, for example, 0.1 to 5 MPa preferably at a temperature of 50 to 170°C, more preferably at a temperature of 60 to 160°C, and further preferably at a temperature of 80 to 160°C. Furthermore, the reaction time for hydrogenation is, for example, 3 to 80 hours.

Thus, Compound (I) can be obtained at a high purity. Compound (I) thus obtained has fragrance notes of citrus and green odors as well as a floral-like odor (especially muguet odor) in good balance.

### [Fragrance Composition]

As described above, the fragrance composition of the present invention contains 4,7-dimethyloctan-3-ol (Compound (I)). The amount of Compound (I) contained in the fragrance composition is preferably 0.01 to 100 % by mass, more preferably 0.1 to 15 % by mass, and further preferably 0.3 to 5 % by mass. When containing 0.01 to 100 % by mass of Compound (I), the fragrance composition can be imparted with a stronger floral feeling.

Moreover, the fragrance composition of the present invention can contain, as a fragrance other than Compound (I), another fragrance component that is commonly used or a formulated perfume with a desired composition. When containing a fragrance other than Compound (I), the fragrance composition of the present invention can be imparted with an odor of, for example, a floral tone, a bouquet tone, a hyacinth tone, a geranium tone, a rose tone, a bergamot tone, an orchid tone, or a lily of the valley tone (muguet).

In the fragrance composition of the present invention, examples of other fragrances that can be used in combination with Compound (I) include alcohols other than Compound (I), hydrocarbons, phenols, esters, carbonates, aldehydes, ketones, acetals, ethers, carboxylic acid, lactones, nitriles, Schiff bases, and fragrance components such as natural essential oils and natural extracts.

Among them, alcohols other than Compound (I), esters, carbonates, aldehydes, ketones, ethers, and lactones are preferable, and particularly, alcohols other than Compound (I) and esters are more preferable.

Examples of alcohols other than Compound (I) include terpene alcohols, aromatic alcohols, and aliphatic alcohols. Among them, terpene alcohols and aromatic alcohols are preferable.

Examples of terpene alcohols include linalool, citronellol geraniol, nerol, terpineol, α-terpineol, dihydromyrcenol, farnesol, nerolidol, cedrol, menthol, and borneol.

Examples of aromatic alcohols include phenylethyl alcohol, benzyl alcohol, dimethyl benzyl carbinol, phenylethyl dimethyl carbinol, and phenyl hexanol.

Examples of aliphatic alcohols include cis-3-hexenol, 1-(2,2,6-trimethylcyclohexyl)-3-hexanol, Amber Core (Trade Name of Kao Corporation, 1-(2-tert-butylcyclohexyloxy)-2-butanol, Sandalmysore Core (Trade Name of Kao Corporation, 2-methyl-4-(2,2,3-trimethyl-3-cyclopenten-1-yl)-2-buten-1-ol), Magnol (Trade Name of Kao Corporation, a mixture containing, as a main component, ethylnorbornylcyclohexanol, Undecavertol (Trade Name of Givaudan Roure K.K., 4-methyl-3-decene-5-ol), and isobornylcyclohexanol.

Examples of hydrocarbons include limonene, α-pinene, β-pinene, terpinene, cedrene, longifolene, and valencene.

Examples of phenols include guaiacol, eugenol, isoeugenol, thymol, p-cresol, vanillin, and ethyl vanillin.

Examples of esters include formate ester, acetate ester, propionate ester, butyrate ester, valerate ester, hexanoate ester, heptanoate ester, nonenoate ester, benzoate ester, cinnamate ester, salicylate ester, brassylate ester, tiglate ester, jasmonate ester, glycidate ester, and anthranilate ester.

Among these esters, acetate ester, propionate ester, salicylate ester, and jasmonate ester are used preferably.

Examples of formate ester include linalylformate, citronellylformate, and geranylformate.

Examples of acetate ester include hexyl acetate, cis-3-hexenyl acetate, linalyl acetate, citronellyl acetate, geranyl acetate, neryl acetate, terpinyl acetate, nopyl acetate, bornyl acetate, isobornyl acetate, acetyl eugenol, acetyl isoeugenol, o-tert-butylcyclohexyl acetate, p-tert-butylcyclohexyl acetate, tricyclodecenyl acetate, benzyl acetate, phenylethyl acetate, styralyl acetate, cinnamyl acetate, dimethylbenzylcarbinyl acetate, phenylethyl phenyl acetate, 3-pentyltetrahydropyran-4-yl acetate, and p-cresyl phenyl acetate.

Examples of propionate ester include citronellyl propionate, tricyclodecenyl propionate, allylcyclohexyl propionate, ethyl 2-cyclohexyl propionate, benzyl propionate, and styralyl propionate.

Examples of butyrate ester include citronellyl butyrate, dimethylbenzylcarbinyl n-butyrate, and tricyclodecenyl isobutyrate.

Examples of valerate ester include methyl valerate, ethyl valerate, butyl valerate, amyl valerate, benzyl valerate, and phenylethyl valerate. Examples of hexanoate ester include methyl hexanoate, ethyl hexanoate, allyl hexanoate, linalyl hexanoate, and citronellyl hexanoate.

Examples of heptanoate ester include methyl heptanoate and allyl heptanoate.

Examples of nonenoate ester include methyl 2-nonenoate, ethyl 2-nonenoate, and ethyl 3-nonenoate.

Examples of benzoate ester include methyl benzoate, benzyl benzoate, and 3,6-dimethyl benzoate.

Examples of cinnamate ester include methyl cinnamate and benzyl cinnamate.

Examples of salicylate ester include methyl salicylate, n-hexyl salicylate, cis-3-hexenyl salicylate, cyclohexyl salicylate, and benzyl salicylate.

Furthermore, examples of brassylate ester include ethylene brassylate.

Examples of tiglate ester include geranyl tiglate, 1-hexyl tiglate, and cis-3-hexenyl tiglate.

Examples of jasmonate ester include methyl jasmonate and methyl dihydrojasmonate.

Examples of glycidate ester include methyl 2,4-dihydroxy-ethylmethylphenyl glycidate and 4-methylphenylethyl glycidate.

Examples of anthranilate ester include methyl anthranilate, ethyl anthranilate, and dimethyl anthranilate.

Furthermore, examples of other esters include methyl atrarate, allyl cyclohexyl glycolate, Fruitate (Trade Name of Kao Corporation, ethyl tricyclo[5.2.1.0^{2.6}]decan-2 carboxylate), Poirenate (Trade Name of Kao Corporation, ethyl 2-cyclohexyl propionate), Peranat (Trade Name of Kao Corporation, 2-methylpentyl 2-methylvalerate), Melusat (Trade Name of Kao Corporation, ethyl 3,5,5-trimethyl hexanoate), and Irotyl (Trade Name of Kao Corporation, ethyl 2-ethylcapronate).

Examples of carbonates include Liffarome (Trade Name of IFF, cis-3-hexenyl methyl carbonate), Jasmacyclat (Trade Name of Kao Corporation, methyl cyclooctyl carbonate), and Floramat (Trade Name of Kao Corporation, ethyl 2-tert-butylcyclohexyl-carbonate) .

Examples of aldehydes include n-octanal, n-nonanal, n-decanal, n-dodecanal, 2-methyl undecanal, 10-undecenal, citronellal, citral, hydroxycitronellal, Triplal (Trade Name of IFF, 2,4-dimethyl-3-cyclohexenyl carboxaldehyde), Cyclovertal (Trade Name of Kao Corporation, dimethyl-3-cyclohexenyl-1-carboxaldehyde), benzaldehyde, phenylacetaldehyde, phenyl propyl aldehyde, cinnamaldehyde, dimethyltetrahydrobenzaldehyde, Bourgeonal (Trade Name of Givaudan, 3-(4-tert-butylphenyl)propanal), Lyral (Trade Name of IFF, hydroxy myrac aldehyde), Pollenal II (Trade Name of Kao Corporation, 2-cyclohexyl propanal), Lilial (Trade Name of Givaudan, p-tert-butyl-α-methylhydrocinnamaldehyde), p-isopropyl-α-methylhydrocinnamaldehyde, Floralozone (Trade Name of IFF, 3-(o- (and p-) ethylphenyl)-2,2-dimethylpropionaldehyde), α-amyl cinnamaldehyde, α-hexyl cinnamaldehyde, hexyl cinnamaldehyde, heliotropin, and Helional (Trade Name of IFF, alpha-methyl-1,3-benzodioxole-5-propanal).

Examples of ketones include α-ionone, β-ionone, γ-ionone, α-methyl ionone, α-methyl ionone, γ-methyl ionone, damascenone, methyl heptenone, 4-methylene-3,5,6,6-tetramethyl-2-heptanone, acetophenone, amyl cyclopentanone, dihydrojasmone, rose ketone, carvone, menthone, camphor, acetyl cedrene, isolongifolanone, nootkatone, benzyl acetone, anisyl acetone, methyl β-naphthyl ketone, 2,5-dimethyl-4-hydroxy-3(2H)-furanone, maltol, muscone, civetone, cyclopentadecanone, Calone (Trade Name of Firmenich, 7-methyl-3,4-dihydro-2H-benzodioxepin-3-one), raspberry ketone, methyl-b-naphthyl ketone, and heliotropyl acetone.

Examples of acetals include acetaldehyde ethylphenylpropyl acetal, citral diethyl acetal, phenylacetaldehyde glyceryl acetal, ethyl acetoacetate ethylene glycol acetal, Boisambrene Forte (Trade Name of Kao Corporation), and Troenan (Trade Name of Kao Corporation).

Examples of ethers include ethyllinalool, cedryl methyl ether, estragole, anethole, β-naphthyl methyl ether, β-naphthyl ethyl ether, limonene oxide, rose oxide, nerol oxide, 1,8-cineole, rose furan, Ambroxan (Trade Name of Kao Corporation, dodecahydro-3a,6,6,9a-tetramethyl naphto[2,1-b]furan), Herbavert (Trade Name of Kao Corporation, 3,3,5-trimethylcyclohexyl ethyl ether), Galaxolide (Trade Name of IFF, hexamethylhexahydrocyclopentabenzopyran), and phenylacetaldehyde dimethyl acetal.

Examples of carboxylic acids include benzoic acid, phenylacetic acid, cinnamic acid, hydrocinnamic acid, butyric acid, and 2-hexenoic acid.

Examples of lactones include γ-decalactone, δ-decalactone, γ-valerolactone, γ-nonalactone, γ-undecalactone, δ-hexalactone, γ-jasmolactone, whisky lactone, coumarin, cyclopentadecanolide, cyclohexadecanolide, ambrettolide, 11-oxahexadecanolide, and butylidenephthalide. Examples of nitriles include geranyl nitrile, citronellyl nitrile, and dodecanenitrile.

Examples of Schiff bases include aurantiol and ligantral. Examples of natural essential oils and natural extracts include orange, lemon, lime, bergamot, vanilla, mandarin, peppermint, spearmint, lavender, chamomile, rosemary, eucalyptus, sage, basil, rose, rockrose, geranium, jasmine, ylang ylang, anise, clove, ginger, nutmeg, cardamon, cedar, cypress, vetiver, patchouli, lemongrass, labdanum, and grapefruit.

The amount of these other fragrances to be contained except for Compound (I) can be selected suitably depending on, for example, the type of the formulated perfume as well as the type and intensity of the intended odor. The amount of each of them contained in the fragrance composition is preferably 0.0001 to 99.99 % by mass, and more preferably 0.001 to 80 % by mass. Furthermore, the total amount of the other fragrances contained in the fragrance composition is preferably 5 to 99.99 % by mass, and more preferably 50 to 99.95 % by mass.

The fragrance composition of the present invention can contain an oil, which itself has no odor, to be used as a base that allows 4,7-dimethyloctan-3-ol (Compound (I)) and the other fragrance materials to be contained therein. Such an oil allows a fragrance component to be mixed uniformly, to be easily mixed into a product, and to easily impart a suitable intensity of fragrance. Specific examples of such an oil include polyhydric alcohols such as ethylene glycol, propylene glycol, butylene glycol, and dipropylene glycol, esters such as isopropyl myristate, dibutyl adipate, and diethyl sebacate, and hydrocarbons such as liquid paraffin and squalane. The amount of such an oil to be contained in the fragrance composition is preferably 1 to 95 % by mass, more preferably 10 to 80 % by mass, and further preferably 15 to 50 % by mass.

Furthermore, the fragrance composition of the present invention may contain a surfactant such as polyoxyethylene alkyl ether or sorbitan fatty acid ester.

### [Use as Fragrance Component]

A fragrance composition containing 4,7-dimethyloctan-3-ol (Compound (I)) of the present invention can be used, as fragrance components of various types of products, as formulated perfumes with a strong fresh floral feeling. Therefore, the present invention provides a method of using, as a fragrance, a fragrance composition containing 4,7-dimethyloctan-3-ol (Compound (I)). For the method of using said compound, it can be contained, alone or in combination with other components, in the bases of toiletry products such as soaps, cosmetics, hair cosmetics, detergents, softeners, spray products, air fresheners, perfumes, and bath agents. Accordingly, the present invention provides a method of using a fragrance composition containing 4,7-dimethyloctan-3-ol (Compound (I)) as a fragrance for toiletry products such as soaps, cosmetics, hair cosmetics, detergents, softeners, spray products, air fresheners, perfumes, and bath agents.

In the aforementioned method of using the fragrance composition, when the fragrance composition contains 4,7-dimethyloctan-3-ol (Compound (I)), the amount of Compound (I) to be used is preferably 0.01 to 100 % by mass, more preferably 0.1 to 15 % by mass, and further preferably 0.3 to 5 % by mass, with respect to the whole fragrance composition. Compound (I) contained in an amount of 0.01 to 100 % by mass can impart a stronger floral feeling.

Particularly, it is preferable that the fragrance composition containing 4,7-dimethyloctan-3-ol (Compound (I)) of the present invention be used to treat hairs or fabrics such as clothing to impart floral-like fragrances. Examples of the products, for which it can be used preferably as a fragrance component, include hair cosmetics such as shampoos and hair conditioners as well as softeners for clothing.

Accordingly, the present invention also provides a cleaner composition containing a fragrance composition of the present invention, a cosmetic containing a fragrance composition of the present invention, a fabric treatment composition containing a fragrance composition of the present invention, a fragrant deodorant containing a fragrance composition of the present invention, and a cleaning nonwoven fabric containing a fragrance composition of the present invention.

The cleaner composition of the present invention is preferably a body cleaner composition, a cleaner composition for clothing, or a cleaner composition for hard surfaces, more preferably a body cleaner composition or a cleaner composition for clothing, and further preferably a cleaner composition for clothing. Furthermore, examples of the cleaner composition of the present invention include a powder cleaner composition and a liquid cleaner composition, and it is preferably a liquid cleaner composition.

Examples of the body cleaner composition include a skin cleaner composition and a hair cleaner composition, and it is preferably a skin cleaner composition.

Examples of the cleaner composition for hard surfaces include an all purpose cleaner and a cleaner composition for tableware.

The fabric treatment composition of the present invention is preferably a softener.

The cosmetic of the present invention is preferably a perfume, a body cosmetic, or a hair cosmetic.

The softener of the present invention contains, for example, quaternary ammonium salt and a fragrance composition of the present invention. The above-mentioned softener may further contain a germicide, a viscosity modifier, a pH adjuster, a solvent, etc.

For the quaternary ammonium salt, any conventionally known one can be used. Examples thereof include octyl trimethyl ammonium chloride, dodecyl trimethyl ammonium chloride, hexadecyl trimethyl ammonium chloride, beef tallow trimethyl ammonium chloride, coconut oil trimethyl ammonium chloride, octyl dimethyl benzyl ammonium chloride, decyl dimethyl benzyl ammonium chloride, dioctadecyl dimethyl ammonium chloride, distearoyloxyethyl dimethyl ammonium chloride, dioleoyloxyethyl dimethyl ammonium chloride, N-stearoyl oxyethyl-N,N-dimethyl-N-(2-hydroxyethy)ammonium methyl sulfate, N,N-distearoyl oxyethyl-N-methyl-N-(2-hydroxyethyDammonium methyl sulfate, N-oleoyloxyethyl-N,N-dimethyl-N-(2-hydroxyethyl)ammonium methyl sulfate, distearyl methylamine hydrochloride, dioleyl methylamine hydrochloride, distearyl methylamine sulfate, N-(3-octadecanoylaminopropyl)-N-(2-octadecanoyloxyethyl-N-methylamine hydrochloride, methyl-1-beef tallow amide ethyl-2-beef tallow alkyl imidazolinium methyl sulfate, methyl-1-hexadecanoyl amide ethyl-2-pentadecyl imidazolinium chloride, ethyl-1-octadecenoyl amide ethyl-2-heptadecenyl imidazolinium ethyl sulfate, 1-octadecanoyl aminoethyl-2-heptadecyl imidazoline hydrochloride, 1-octadecenoyl aminoethyl-2-heptadecenyl imidazoline hydrochloride, and N,N-di[2-(alkanoyloxy)-ethyl]-N-(2-hydroxyethyl)-N-methyl ammonium sulfate. One of these can be used alone or two or more of them can be used in combination.

Examples of the above-mentioned germicide include alcohols having 1 to 8 carbon atoms, benzoic acids, and phenols. Specific examples thereof include ethanol, propylene glycol, benzyl alcohol, salicylic acid, methyl p-hydroxybenzoate, and cresol.

Inorganic or organic salts (excluding a quaternary ammonium salt) can be used as the viscosity modifier mentioned above. Specific examples thereof include sodium chloride, potassium chloride, calcium chloride, magnesium chloride, aluminum chloride, sodium sulfate, magnesium sulfate, potassium sulfate, sodium nitrate, magnesium nitrate, sodium p-toluenesulfonate, sodium glycolate, sodium acetate, potassium acetate, potassium glycolate, and sodium lactate. Preferably, the viscosity modifier is calcium chloride or magnesium chloride.

The softener of the present invention contains water, and generally the remainder of the composition is water. The water is preferably ion exchanged water or distilled water. Preferably, the softener of the present invention has a pH of 1.5 to 6 at 20°C. From the viewpoints of antisepsis/bactericidal activity, the lower the pH is, the better. However, an excessively low pH may cause decomposition of components that are generally mixed into compositions. Therefore, the pH is more preferably 1.5 to 5 and further preferably 2 to 4.5.

Any inorganic or organic acid and alkali can be used for adjusting the pH of the softener of the present invention.

Furthermore, known components that usually are mixed in a softener as other optional components in addition to the above-mentioned components can be mixed in the softener of the present invention in a range that does not hinder the effects of the present invention. Examples of the optional components that can be mixed include: higher fatty acids such as stearic acid, oleic acid, and palmitic acid or esters thereof formed with lower alcohols; nonionic surfactants such as fatty acid glycerol ester, which is an ester of, for example, stearic acid and glycerol; higher alcohols such as stearyl alcohol, palmityl alcohol, and oleyl alcohol; and low-temperature stabilizers such as ethylene glycol and glycerol. In addition to these, for example, urea, pigments, a cellulose derivative, an ultraviolet absorber, and a fluorescent brightener can be mixed.

It is preferable that the cleaner composition of the present invention contain an anionic surfactant other than a fragrance composition containing 4,7-dimethyloctan-3-ol. Furthermore, a nonionic surfactant, a pH adjuster, a viscosity modifier, a solvent, an oil, a preservative, water, etc. can be mixed thereinto.

In the perfume of the present invention, a solvent, water, etc. can be mixed thereinto other than a fragrance composition containing 4,7-dimethyloctan-3-ol.

The present invention provides 4,7-dimethyloctan-3-ol (Compound (I)). The 4,7-dimethyloctan-3-ol (Compound (I)) has citrus and green odors as well as a floral-like odor (especially muguet odor) in good balance as described above. Therefore, the present invention is a method of using 4,7-dimethyloctan-3-ol (Compound (I)) as a fragrance component. Furthermore, the present invention provides a method of using 4,7-dimethyloctan-3-ol (Compound (I)) as a fragrance component.

With respect to the above-mentioned embodiment, the present invention further discloses the following fragrance composition, production method thereof, method of using the fragrance composition, and use thereof.
<1> A fragrance composition containing 4,7-dimethyloctan-3-ol.
<2> The fragrance composition according to <1>, wherein the amount of 4,7-dimethyloctan-3-ol contained therein is preferably 0.01 to 100 % by mass, more preferably 0.1 to 15 % by mass, and further preferably 0.3 to 5 % by mass.
<3> The fragrance composition according to <1> or <2>, further containing at least one selected from alcohols other than 4,7-dimethyloctan-3-ol, esters, carbonates, aldehydes, ketones, ethers, and lactones.
<4> The fragrance composition according to <3>, wherein the esters are at least one selected from the group consisting of acetate ester, propionate ester, salicylate ester, jasmonate ester, methyl atrarate, Fruitate, and Poirenate.
<5> The fragrance composition according to any one of<1> to <4>, wherein the carbonates are Liffarome.
<6> The fragrance composition according to any one of <3> to <5>, wherein the aldehydes are at least one selected from the group consisting of Triplal, Bourgeonal, Lilial, Floralozone, hexyl cinnamaldehyde, and Helional.
<7> The fragrance composition according to any one of<3> to <6>, wherein the ketones are at least one selected from the group consisting of β-ionone, damascenone, muscone, Calone, raspberry ketone, methyl-b-naphthyl ketone, and heliotropyl acetone.
<8> The fragrance composition according to any one of<3> to <7>, wherein the ethers are at least one selected from the group consisting of ethyllinalool, rose oxide, Ambroxan, Galaxolide, and phenylacetaldehyde dimethyl acetal.
<9> The fragrance composition according to any one of<3> to <8>, wherein the lactones are ambrettolide.
<10> The fragrance composition according to any one of<3> to <9>, wherein the alcohols fragrance other than 4,7-dimethyloctan-3-ol are at least one selected from the group consisting of terpene alcohol and aliphatic alcohol.
<11> The fragrance composition according to any one of <1> to <10>, further containing an oil.
<12> The fragrance composition according to any one of <1> to <11>, further containing a surfactant.
<13> A fabric treatment composition (preferably a softener) containing a fragrance composition according to any one of <1> to <12>.
<14> The fabric treatment composition (preferably a softener) according to <13>, further containing a quaternary ammonium salt, a germicide, a viscosity modifier, a pH adjuster, and/or a solvent.
<15> A cosmetic (preferably a perfume, a body cosmetic, or a hair cosmetic) containing a fragrance composition according to any one of <1> to <12>.
<16> A cleaner composition (preferably a body cleaner composition (for example, a skin cleaner composition or a hair cleaner composition), a cleaner composition for clothing, or a cleaner composition for hard surfaces (for example, an all purpose cleaner or a cleaner composition for tableware), more preferably a body cleaner composition or a cleaner composition for clothing, and further preferably a cleaner composition for clothing) containing a fragrance composition according to any one of <1> to <12>.
<17> A cleaner composition (preferably a powder cleaner composition or a liquid cleaner composition, more preferably a liquid cleaner composition) containing a fragrance composition according to any one of <1> to <12>.
<18> A fragrant deodorant containing a fragrance composition according to any one of <1> to <12>.
<19> A cleaning nonwoven fabric containing a fragrance composition according to any one of <1> to <12>.
<20> A method of producing 4,7-dimethyloctan-3-ol, including a step of carrying out a cross aldol reaction using isovaleraldehyde and 3-pentanone, and then dehydrating and reducing to produce 4,7-dimethyloctan-3-ol.
<21> 4,7-dimethyloctan-3-ol.
<22> Use of a fragrance composition according to any one of <1> to <12>, as a fragrance component.
<23> A method of using 4,7-dimethyloctan-3-ol as a fragrance component.

### Examples

Details of the measurement methods carried out in the following production examples and the like are described together below.

### [Compound Identification]

The structure of each compound obtained in the following production examples and the like was identified by a nuclear magnetic resonance spectrum (¹H-NMR). The nuclear magnetic resonance spectrum was measured using chloroform-d as a solvent, by "Mercury 400" (product name) manufactured by Varian.

### [Production Example 1]

### Production of 4,7-methyloct-4-en-3-one (Compound (II))

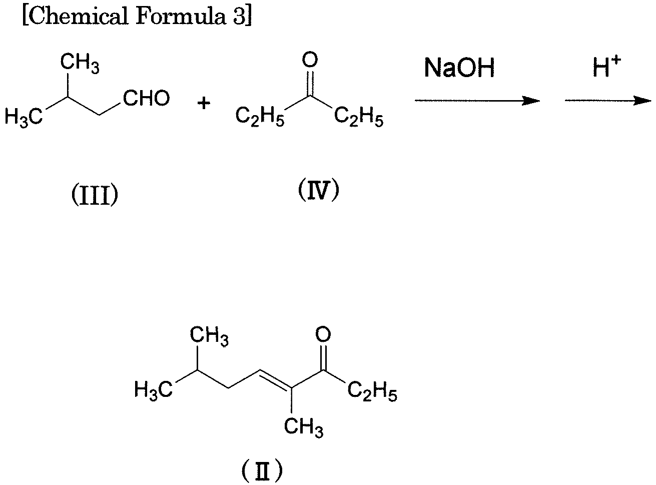

In a flask, sodium hydroxide (0.1 g), water (20 g), and 3-pentanone (IV) (50 g, 0.58 mole) were placed and maintained at 15°C, and isovaleraldehyde (III) (20 g, 0.23 mole) was dropped in five hours while stirring. Then sodium hydroxide (0.2 g) was added thereto additionally, which was further stirred at 25°C for 48 hours. After stirring, the reaction solution was allowed to stand still and then the lower layer was removed. Thereafter, excess 3-pentanone (IV) was removed from the upper layer by distillation.

Subsequently, 85% phosphoric acid (1 g) was added to the upper layer portion of the reaction solution. With a water fractionator being attached to the flask, it was heated to 120°C to be dehydrated. Sodium hydroxide (0.7 g) was added to the reaction solution thus dehydrated to neutralize it and then magnesium sulfate was added to dry it. Thereafter, the magnesium sulfate was removed by filtration. The reaction solution thus obtained was concentrated and thereby a residue (23 g) was obtained.

The residue thus obtained was purified using a silica gel column (Eluent: Hexane : Ethyl acetate = 99:1 (v/v)). The purified product thus obtained was further purified by distillation, and thereby 20 g of Compound (II) was obtained.

Compound (II) thus obtained had a purity of 97%.

### [Production Example 2]

### Production of 4,7-dimethyloctan-3-ol (Compound (I))

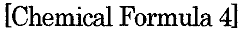

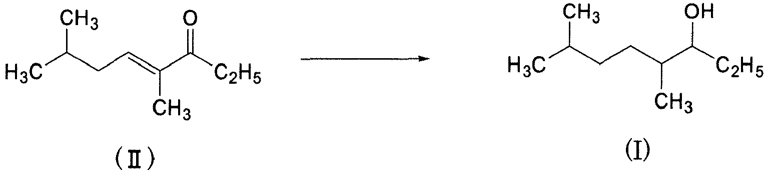

In a pressure resistant vessel for hydrogenation, 5% active carbon supporting ruthenium catalyst (0.06 g), isopropyl alcohol (1.0g), and Compound (II) (1.0g) obtained in Production Example 1 were placed and then were hydrogenated at 90°C under a hydrogen pressure of 0.5 MPa for 18 hours. The reaction solution thus obtained was filtered and thereby 2 g of filtrate was obtained (with a yield of 95%).

The filtrate thus obtained was concentrated and the concentrated solution thus obtained was purified by distillation and thereby 0.8 g of Compound (I) was obtained. Compound (I) thus obtained had a purity of 99%.

With respect to Compound (I), measurement results of ¹H-NMR are shown below.
¹H-NMR (CDCl₃, 400 MHz, δ ppm): 0.80-0.90 (m, 9H), 0.96 (dt, J=7.2 Hz, 5.2 Hz, 3H), 1.05-1.60 (m, 9H), 3.30-3.45 (m, 1H)

### [Comparative Production Example 1]

### Production of 2,5-dimethylhexane-1-ol (also referred to as "Compound (VI)" in the present description)

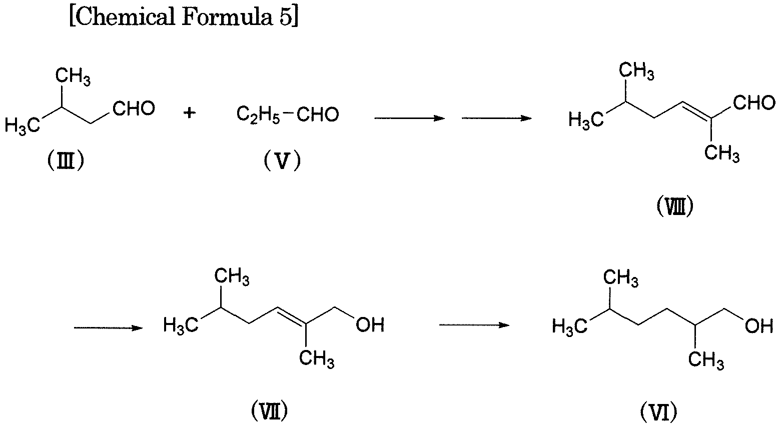

In a flask, piperidine (0.9 g), heptanoic acid (1.4 g), and isovaleraldehyde (III) (20 g) were placed and propionaldehyde (V) (10 g) was dropped at 75°C in three hours while stirring. The reaction solution thus obtained was cooled, which was then separated into layers and the lower layer was removed. Thereafter, acetic acid (1 g) and water (3 g) were added to the upper layer to neutralize it. Magnesium sulfate was added to the upper layer thus obtained to dry it and then the magnesium sulfate was removed by filtration. The reaction solution thus obtained was concentrated and thereby 2,5-dimethylhex-2-enal (referred to as "Compound (VIII)" in the present description) (20 g) was obtained (with the yield of Compound (VIII) being 66%).

In a flask, methanol (20 g), 50% aqueous sodium hydroxide solution (0.2 g), water (8 g), and sodium borohydride (2 g) were placed and Compound (VIII) (20 g) was dropped thereinto at 15°C in 0.5 hour, which was further stirred for 0.5 hour. Then, 6N sulfuric acid aqueous solution (20 mL) was added to the reaction solution to decompose excess sodium borohydride. Further 50% aqueous sodium hydroxide solution (5 g) was added thereto and thereby the reaction solution was neutralized. Thereafter, methanol was evaporated from the reaction solution at atmospheric pressure and then magnesium sulfate was added to the residual liquid to dry it. Thereafter, the magnesium sulfate was removed by filtration. The residual liquid thus obtained was concentrated and thereby 14 g of a concentrated solution was obtained (with the yield of 2,5-dimethylhex-2-ene-1-ol being 81%). The concentrated solution thus obtained was purified by a silica gel column (Eluent: Hexane : Ethyl acetate = 97 : 3 (v/v)) and thereby 2,5-dimethylhex-2-ene-1-ol (referred to as "Compound (VII)" in the present description) (1.4 g) was obtained. Compound (VII) thus obtained had a purity of 98%.

In a pressure resistant vessel for hydrogenation, 5% active carbon supporting ruthenium catalyst (0.02 g), isopropyl alcohol (1.2 g), and Compound (VII) (0.3 g) were placed and then were hydrogenated at 90°C under a hydrogen pressure of 0.5 MPa for three hours. The reaction solution thus obtained was filtered and thereby a filtrate (0.3 g) was obtained (with a yield of 88%).

The filtrate thus obtained was concentrated and the concentrated solution thus obtained was purified by distillation. Thus, 0.1g of 2,5-dimethylhexane-1-ol (VI) was obtained. Compound (VI) thus obtained had a purity of 100%.

With respect to Compound (VI), measurement results of ¹H-NMR are shown below.
¹H-NMR (CDCl₃, 400 MHz, δ ppm): 0.88 (d, J=6.4 Hz, 3H), 0.88 (d, J=6.4 Hz, 3H), 0.92 (d, J=6.4 Hz, 3H), 1.02-1.30 (m, 4H), 1.32-1.64 (m, 3H), 3.38-3.54 (m, 2H).

### [Comparative Production Example 2]

### Production of 2,5-dimethyl-2-ethylhexane-1-ol (also referred to as "Compound (XI)" in the present description)

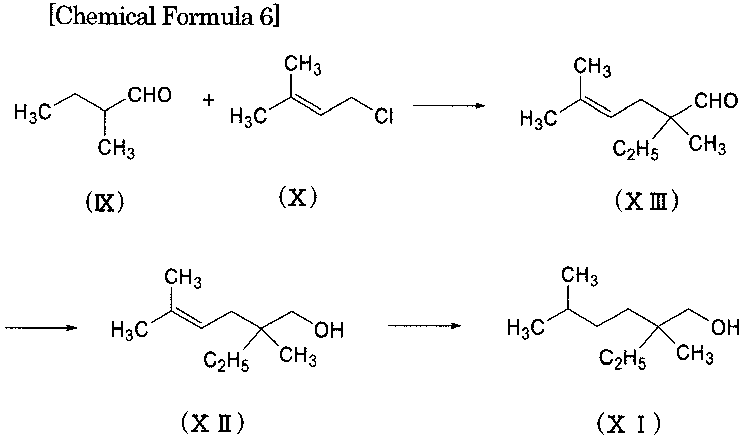

In a flask, toluene (20 mL), sodium hydroxide (10 g), butylammonium iodide (TBAI) (1.3 g), and water (10 mL) were added and 2-methylbutanal (IX) (21 g) and 1-chloro-3-methyl-2-butene (X) (31 g) were dropped at 70°C in one hour. Thereafter, this was separated into layers using water and ether. The upper layer (an organic layer) was washed with an aqueous sodium sulfite solution and then magnesium sulfate was added thereto to dry it. Thereafter, the magnesium sulfate was removed by filtration. The organic layer thus obtained was concentrated and thereby 2-ethyl-2,5-dimethylhexyl-4-enal (also referred to as Compound (XIII) in the present description) (42 g) was obtained.

In a flask, methanol (5 g), 50% aqueous sodium hydroxide solution (1 g), water (1 g), and sodium borohydride (0.5 g) were placed and Compound (XIII) (5 g) was dropped thereinto at 15°C in 0.5 hour, which was then stirred for one hour. Then, 6N sulfuric acid aqueous solution was added to the reaction solution thus obtained to decompose excess sodium borohydride. Further 50% aqueous sodium hydroxide solution was added thereto and thereby the reaction solution was neutralized. Thereafter, methanol was evaporated from the reaction solution at atmospheric pressure. Magnesium sulfate was added to the residual liquid thus obtained to dry it and then the magnesium sulfate was removed by filtration. The residual liquid thus obtained was concentrated and thereby 3.8 g of a concentrated solution was obtained (with the yield of 2-ethyl-2,5-dimethylhex-4-ene-1-ol being 63%). The concentrated solution thus obtained was purified by a silica gel column (Eluent: Hexane : Ethyl acetate = 97: 3 (v/v)) and thereby 2-ethyl-2,5-dimethylhex-4-ene-1-ol (referred to as "Compound (XII)" in the present description) (2 g) was obtained. Compound (XII) had a purity of 100%.

In a pressure resistant vessel for hydrogenation, 5% active carbon supporting ruthenium catalyst (0.03 g), isopropyl alcohol (1.2 g), and Compound (XII) (0.5 g) were placed and then were hydrogenated at 90°C under a hydrogen pressure of 0.5 MPa for 2.5 hours. The reaction solution thus obtained was filtered and thereby a filtrate (0.83 g) was obtained (with a yield of 99%).

The filtrate thus obtained was concentrated and the concentrated solution thus obtained was purified by distillation. Thus, 0.5 g of Compound (XI) was obtained. Compound (XI) thus obtained had a purity of 100%.

With respect to Compound (XI), measurement results of ¹H-NMR are shown below.
¹H-NMR (CDCl₃, 400 MHz, δ ppm): 0.81 (t, J=7.6 Hz, 3H), 0.81 (s, 3H), 0.89 (d, J=6.8 Hz, 6H), 1.05-1.35 (m, 6H), 1.35-1.50 (m, 1H), 3.34 (d, J=6.0 Hz, 2H).

### [Odor Evaluation of Fragrance Material]

Odor evaluation of Compound (I) obtained in Production Example 2 was carried out in accordance with the following odor evaluation method.

One expert who had an experience of seven years of blending odors and evaluating fragrances determined the fragrance note by a smelling strip method. About 5 mm of the end of each smelling strip (fragrance test paper with a width of 6 mm and a length of 150 mm) was immersed in a sample and thereby evaluation was performed.

With respect to the odor, fragrances that are sensed mainly (main odors) were listed from the strongest to the weakest and further fragrances that are sensed secondarily (secondary odors) were also listed. Furthermore, the presence or absence of a floral feeling was also determined.

Instead of Compound (I) obtained in Production Example 2, odor evaluation was carried out using Compound (VI) obtained in Comparative Production Example 1, Compound (XI) obtained in Comparative Production Example 2, and 3,7-dimethyloctane-3-ol (Tetrahydrolinalool represented by Formula (XIV) below (also referred to as Compound (XIV) in the present description) in the same manner as in the evaluation of Compound (I) obtained in Production Example 2.

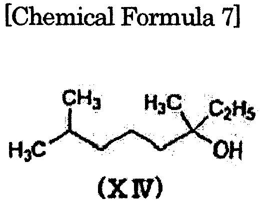

**[Table 1]**

| Fragrance Material | Production Example 2 | Comparative Production Example 1 | Comparative Production Example 2 | Comparative Production Example 3 |
|---|---|---|---|---|
| Compound | Compound (I) | Compound (VI) | Compound (XI) | Compound (XIV) |
| Presence or Absence of Green Feeling | Present | Absent | Absent | Absent |
| Main Odor | Floral | Herbal | Woody | Floral |
| Secondary Odor | Citrus | Woody | Herbal | Herbal |
| | Green | Citrus | Citrus | - |

As shown in Table 1 above, it was confirmed that Compound (I) of the present invention had a floral-like main odor, and in addition, citrus and green odors in good balance.

### Example 1 (Softener)

Compound (I) obtained in Production Example 2 was used to prepare a softener having the composition shown in Table 2. With respect to this softener, the following odor evaluation was carried out.

### [Odor Evaluation]

One expert who had an experience of seven years of blending odors and evaluating fragrances evaluated odors in the following manner. In a 100ml glass bottle, 50 ml of the softener having the composition shown in Table 2 was placed, which was then sealed and allowed to stand at 25°C for 24 hours. Thereafter, the expert evaluated the odor at the mouth of the bottle when it was opened.

With respect to the odor, fragrances that were sensed mainly (main odors) were listed sequentially from the strongest, and the assessment was also noted.

**[Table 2]**

| (% by mass) | | |
|---|---|---|
| | | Example 1 |
| Softener | N,N-di[2-(alkanoyloxy)ethyl]-N-(2-hydroxyethyl)-N-methyl ammonium sulfate | 15 |
| | Stearic acid | 0.5 |
| | Calcium chloride | 0.05 |
| | Ethanol | 2 |
| | Fragrance composition | 0.2 |
| | Ion exchanged water | Suitable amount |
| | pH Adjuster | Suitable amount |
| | Total % by mass | 100 |
| | pH | 4 |
| | Amount of Compound (I) contained in fragrance composition % by mass | 100 |
| Evaluation | Main Odor | Floral |
| | | Citrus |
| | | Green |
| | Assessment | Excellent fresh feeling |

As shown in Table 2 above, it was confirmed that the softener containing the fragrance composition of the present invention allowed a fresh, strong floral feeling to be obtained.

### Example 2 and Comparative Examples 1 to 4 (Fragrance compositions for perfumes)

Compound (I) obtained in Production Example 2 was used to prepare a fragrance composition for perfumes having the composition shown in Table 3 (Example 2). Furthermore, as comparative examples, a fragrance composition not including Compound (I) (Comparative Example 1), Compound (VI) obtained in Comparative Production Example 1 (Comparative Example 2), Compound (XI) obtained in Comparative Production Example 2 (Comparative Example 3), and Compound (XIV) (Comparative Example 4) were used to produce fragrance compositions for perfumes, each having the composition shown in Table 3. Odor evaluation of the fragrance compositions was carried out by the same method as in the evaluation of Compound (I) obtained in Production Example 2 and the odors obtained when the compositions were used for perfumes were assessed by sensory evaluation. The results of the sensory evaluation are shown in Table 4.

**[Table 3]**

| (parts by mass) | | | | | |
|---|---|---|---|---|---|
| | Comparative Example 1 | Example 2 | Comparative Example 2 | Comparative Example 3 | Comparative Example 4 |
| cis-3-Hexenol | 2 | 2 | 2 | 2 | 2 |
| cis-3-Hexenyl salicylate | 50 | 50 | 50 | 50 | 50 |
| Liffarome¹⁾ | 2 | 2 | 2 | 2 | 2 |
| Styralyl acetate | 10 | 10 | 10 | 10 | 10 |
| Calone²⁾ | 0.5 | 0.5 | 0.5 | 0.5 | 0.5 |
| Floralozone³⁾ | 5 | 5 | 5 | 5 | 5 |
| Helional⁴⁾ | 50 | 50 | 50 | 50 | 50 |
| Damascenone | 1 | 1 | 1 | 1 | 1 |
| Geraniol | 50 | 50 | 50 | 50 | 50 |
| Phenyl hexanol | 20 | 20 | 20 | 20 | 20 |
| Benzyl acetate | 50 | 50 | 50 | 50 | 50 |
| Methyl dihydrojasmonate | 200 | 200 | 200 | 200 | 200 |
| Bourgeonal⁵⁾ | 10 | 10 | 10 | 10 | 10 |
| Ethyllinalool | 70 | 70 | 70 | 70 | 70 |
| Lilial⁶⁾ | 100 | 100 | 100 | 100 | 100 |
| α-Terpineol | 50 | 50 | 50 | 50 | 50 |
| β-Ionone | 5 | 5 | 5 | 5 | 5 |
| Amber Core⁷⁾ | 50 | 50 | 50 | 50 | 50 |
| Sandalmysore Core⁸⁾ | 5 | 5 | 5 | 5 | 5 |
| Methyl atrarate | 2 | 2 | 2 | 2 | 2 |
| Ambrettolide | 2 | 2 | 2 | 2 | 2 |
| Muscone | 2 | 2 | 2 | 2 | 2 |
| Compound (I) | - | 50 | - | - | - |
| Compound (VI) | - | - | 50 | - | - |
| Compound (XI) | - | - | - | 50 | - |
| Compound (XIV) | - | - | - | - | 50 |
| Dipropylene glycol | 263.5 | 213.5 | 213.5 | 213.5 | 213.5 |

| | | | | | |
|---|---|---|---|---|---|
| [0145] 1) Liffarome: IFF (Trade Name), Compound Name: cis-3-hexenyl methyl carbonate, 2) Calone: Firmenich (Trade Name), Compound Name: 7-methyl-3,4-dihydro-2H-benzodioxepin-3-one, 3) Floralozone: IFF (Trade Name), Compound Name: 3-(o- (and p-)ethylphenyl)-2,2-dimethylproprionaldehyde, 4) Helional: IFF (Trade Name), Compound Name: alpha-methyl-1,3-benzodioxole-5-propanal, 5) Bourgeonal: Givaudan (Trade Name), Compound Name: 3-(4-tert-butylphenyl)propanal, 6) Lilial: Givaudan (Trade Name), Compound Name: p-tert-butyl-α-methylhydrocinnamaldehyde, 7) Amber Core: Kao Corporation (Trade Name), Compound Name: 1-(2-tert-butyl cyclohexyloxy)-2-butanol, 8) Sandalmysore Core: Kao Corporation (Trade Name), Compound Name: 2-methyl-4-(2,2,3-trimethyl-3-cyclopenten-1-yl)-2-buten-1-ol | | | | | |

**[Table 4]**

| | |
|---|---|
| Comparative Example 1 | A fresh and sweet aquatic-floral like odor |
| Example 2 | An odor reminding of muguet and with a feeling of cleanliness and freshness |
| Comparative Example 2 | Herbal-like grassy-smelling was added, and a floral feeling was impaired. |
| Comparative Example 3 | Woody-like bitterness was added, and a floral feeling was impaired. |
| Comparative Example 4 | A floral feeling and sweetness were present, but freshness was insufficient. |

As shown in Table 4 above, it was confirmed that the fragrance composition of the present invention was able to obtain a floral tone with a feeling of cleanliness and freshness by containing additional fragrance components.

### Example 3 and Comparative Examples 5 to 8 (Fragrance compositions for liquid detergents for clothing)

Compound (I) obtained in Production Example 2 was used to prepare a white floral-type fragrance composition for liquid detergents for clothing having the composition shown in Table 5. Furthermore, as comparative examples, a fragrance composition not including Compound (I) (Comparative Example 5), Compound (VI) obtained in Comparative Production Example 1 (Comparative Example 6), Compound (XI) obtained in Comparative Production Example 2 (Comparative Example 7), and Compound (XIV) (Comparative Example 8) were used to produce white floral-type fragrance compositions for liquid detergents for clothing, each having the composition shown in Table 5. Odor evaluation was carried out by the same method as in the evaluation of Compound (I) obtained in Production Example 2 and the odors obtained when the compositions were used for detergents were assessed by sensory evaluation. The results of the sensory evaluation are shown in Table 6.

**[Table 5]**

| (parts by mass) | | | | | |
|---|---|---|---|---|---|
| | Comparative Example 5 | Example 3 | Comparative Exemple 6 | Comparative Example 7 | Comparative Example |
| Dihydromyrcenol | 50 | 50 | 50 | 50 | 50 |
| Triplal¹⁾ | 4 | 4 | 4 | 4 | 4 |
| Magnol²⁾ | 2 | 2 | 2 | 2 | 2 |
| Undecavertol³⁾ | 2 | 2 | 2 | 2 | 2 |
| o-t-Butyl cyclohexyl acetate | 20 | 20 | 20 | 20 | 20 |
| Poirenate⁴⁾ | 2 | 2 | 2 | 2 | 2 |
| Raspberry ketone | 5 | 5 | 5 | 5 | 5 |
| Fruitate⁵⁾ | 2 | 2 | 2 | 2 | 2 |
| Tricyclodecenyl propionate | 100 | 100 | 100 | 100 | 100 |
| Geraniol | 100 | 100 | 100 | 100 | 100 |
| Rose oxide | 1 | 1 | 1 | 1 | 1 |
| Hexyl cinnamaldehyde | 100 | 100 | 100 | 100 | 100 |
| Methyl dihydrojasmonate | 50 | 50 | 50 | 50 | 50 |
| Dimethyl anthranilate | 5 | 5 | 5 | 5 | 5 |
| Methyl-b-naphthyl ketone | 5 | 5 | 5 | 5 | 5 |
| Lilial⁶⁾ | 100 | 100 | 100 | 100 | 100 |
| Linalool | 50 | 50 | 50 | 50 | 50 |
| Terpineol | 50 | 50 | 50 | 50 | 50 |
| Phenylacetaldehyde dimethylacetal | 4 | 4 | 4 | 4 | 4 |
| Amber Core⁷⁾ | 30 | 30 | 30 | 30 | 30 |
| Isobornylcyclohexanol | 20 | 20 | 20 | 20 | 20 |
| Galaxolide50%IPM⁸⁾ | 60 | 60 | 60 | 60 | 60 |
| Ambroxan⁹⁾5%DPG¹⁰⁾ | 4 | 4 | 4 | 4 | 4 |
| Heliotropyl acetone | 1 | 1 | 1 | 1 | 1 |
| Compound (I) | - | 50 | - | - | - |
| Compound (VI) | - | - | 50 | - | - |
| Compound (XI) | - | - | - | 50 | - |
| Compound (XIV) | - | - | - | - | 50 |
| Dipropylene glycol | 233 | 183 | 183 | 183 | 183 |

| | | | | | |
|---|---|---|---|---|---|
| [0150] 1) Triplal: IFF (Trade Name), Compound Name: 2,4-dimethyl-3-cyclohexene-1-carboxaldehyde, 2) Magnol: Kao Corporation (Trade Name), a mixture containing ethyl norbornyl cyclohexanol as a main component, 3) Undecavertol: Givaudan Roure K.K. (Trade Name), Compound Name: 4 -methyl-3-decene- 5-ol, 4) Poirenate: Kao Corporation (Trade Name), Compound Name: ethyl 2-cyclohexyl propionate, 5) Fruitate: Kao Corporation (Trade Name), Compound Name: ethyl tricyclo [5.2.1.0^{2.6}] decan-2-carboxylate), 6) Lilial: Givaudan (Trade Name), Compound Name: p-tert-butyl-α-methylhydrocinnamaldehyde, 7) Amber Core: Kao Corporation (Trade Name), Compound Name: 1-(2-tert-butyl cyclohexyloxy)-2-butanol, 8) 50%IPM: indicating a 50% isopropyl myristate (IPM) solution, 9) Ambroxan: Kao Corporation (Trade Name), Compound Name: dodecahydro-3a,6,6,9a-tetramethyl naphto[2,1-b]furan, 10) DPG: Dipropylene glycol. | | | | | |

**[Table 6]**

| | |
|---|---|
| Comparative Example 5 | A bright white floral-like odor |
| Example 3 | A bright odor with a fresh feeling and a feeling of cleanliness |
| Comparative Example 6 | A floral feeling was weak and no effect was observed. |
| Comparative Example 7 | A floral feeling was weak and no effect was observed. |
| Comparative Example 8 | A floral feeling was present, but freshness was insufficient. |

As shown in Table 6 above, it was confirmed that the fragrance composition for liquid detergents for clothing of the present invention was able to obtain a fresh floral tone with a feeling of cleanliness.

0.5 part by mass of the fragrance composition of Example 3 shown in Table 5 was blended into 99.5 parts by mass of a liquid detergent composition having the composition shown in Table 7 and thereby a liquid detergent composition for clothing was obtained. 20 g of this composition was weighed and then was dissolved in 30 L of water. A cotton towel (2 kg) was placed in this water solution and then was laundered for five minutes, was rinsed for one minute, and thereafter was spin-dried. The odor of this cotton towel was evaluated. In the same manner, with respect to Comparative Examples 5 to 8, odors obtained after spin-dry were evaluated. The results of the sensory evaluation are shown in Table 8.

**[Table 7]**

| Ingredients | % by mass |
|---|---|
| Alkyl(C₁₂ -C₁₄)O(EO)₇(PO)₂(EO)₃H | 35 |
| Sodium linear alkyl(C₁₀-C₁₈) benzenesulfonate | 1 |
| Fatty acid (lauric acid and myristic acid) | 1 |
| Monoethanolamine | 5 |
| Sodium sulfite | 0.1 |
| Ethanol | 2 |
| Enzyme | 0.5 |
| Water | Balance |
| Total | 99.5 |

**[Table 8]**

| | |
|---|---|
| Comparative Example 5 | A bright floral odor |
| Example 3 | A natural floral odor with increased freshness as compared with Comparative Example 5 |
| Comparative Example 6 | A floral feeling was weak and there was no difference from the blank (Comparative Example 5). |
| Comparative Example 7 | A floral feeling was weak and there was no difference from the blank (Comparative Example 5). |
| Comparative Example 8 | A floral feeling was slightly stronger than that of the blank (Comparative Example 5). |

As shown in Table 8 above, it was confirmed that the liquid detergent composition of the present invention had a high diffusibility, which resulted in freshness, and thereby provided a natural floral odor with a feeling of cleanliness.

### Industrial Applicability

Since the fragrance composition of the present invention contains 4,7-dimethyloctan-3-ol (Compound (I)), it can be used as a fragrance material having a strong fresh floral fragrance note. Further, since the 4,7-dimethyloctan-3-ol of the present invention has citrus and green odors as well as a floral-like odor in good balance, it can be used alone or in combination with another component, as a fragrance component of a toiletry product such as a hair cosmetic and a softener.

## Claims

1. A fragrance composition comprising 4,7-dimethyloctan-3-ol.

2. The fragrance composition according to claim 1, wherein the amount of 4,7-dimethyloctan-3-ol contained therein is 0.01 to 100 % by mass.

3. The fragrance composition according to claim 1 or 2, wherein the amount of 4,7-dimethyloctan-3-ol contained therein is 0.1 to 15 % by mass.

4. The fragrance composition according to any one of claims 1 to 3, wherein the amount of 4,7-dimethyloctan-3-ol contained therein is 0.3 to 5 % by mass.

5. The fragrance composition according to any one of claims 1 to 4 further comprising at least one selected from the group consisting of alcohol other than 4,7-dimethyloctan-3-ol, ester, carbonate, aldehyde, ketone, ether, and lactone.

6. The fragrance composition according to any one of claims 1 to 5 further comprising an oil.

7. The fragrance composition according to any one of claims 1 to 6 further comprising a surfactant.

8. A fabric treatment composition comprising a fragrance composition according to any one of claims 1 to 7.

9. A softener comprising a fragrance composition according to any one of claims 1 to 7.

10. A cosmetic comprising a fragrance composition according to any one of claims 1 to 7.

11. A cleaner composition comprising a fragrance composition according to any one of claims 1 to 7.

12. A method of using 4,7-dimethyloctan-3-ol as a fragrance component.

13. A method of producing 4,7-dimethyloctan-3-ol, comprising a step of carrying out a cross aldol reaction using isovaleraldehyde and 3-pentanone, and then dehydrating and reducing to produce 4,7-dimethyloctan-3-ol.

14. 4,7-dimethyloctan-3-ol.

## Patentansprüche

1. Duftstoffzusammensetzung, enthaltend 4,7-Dimethyloctan-3-ol.

2. Duftstoffzusammensetzung gemäß Anspruch 1, worin die Menge von 4,7-Dimethyloctan-3-ol, die darin enthalten ist, 0,01 bis 100 Masse-% ist.

3. Duftstoffzusammensetzung gemäß Anspruch 1 oder 2, worin die Menge von 4,7-Dimethyloctan-3-ol, die darin enthalten ist, 0,1 bis 15 Masse-% ist.

4. Duftstoffzusammensetzung gemäß einem der Ansprüche 1 bis 3, worin die Menge von 4,7-Dimethyloctan-3-ol, die darin enthalten ist, 0,3 bis 5 Masse-% ist.

5. Duftstoffzusammensetzung gemäß einem der Ansprüche 1 bis 4, weiterhin enthaltend zumindest eines, ausgewählt aus der Gruppe, bestehend aus einem anderen Alkohol als 4,7-Dimethyloctan-3-ol, Ester, Carbonat, Aldehyd, Keton, Ether und Lacton.

6. Duftstoffzusammensetzung gemäß einem der Ansprüche 1 bis 5, weiterhin enthaltend ein Öl.

7. Duftstoffzusammensetzung gemäß einem der Ansprüche 1 bis 6, weiterhin enthaltend ein Tensid.

8. Textilbehandlungszusammensetzung, enthaltend eine Duftstoffzusammensetzung gemäß einem der Ansprüche 1 bis 7 .

9. Weichmacher, enthaltend eine Duftstoffzusammensetzung gemäß einem der Ansprüche 1 bis 7.

10. Kosmetikum, enthaltend eine Duftstoffzusammensetzung gemäß einem der Ansprüche 1 bis 7.

11. Reinigungszusammensetzung, enthaltend eine Duftstoffzusammensetzung gemäß einem der Ansprüche 1 bis 7.

12. Verfahren zur Verwendung von 4,7-Dimethyloctan-3-ol als Duftstoffkomponente.

13. Verfahren zur Erzeugung von 4,7-Dimethyloctan-3-ol, enthaltend einen Schritt zur Durchführung einer Kreuz-Aldol-Reaktion unter Verwendung von Isovaleraldehyd und 3-Pentanon und anschließendes Dehydratisieren und Reduzieren, zur Erzeugung von 4,7-Dimethyloctan-3-ol.

14. 4,7-Dimethyloctan-3-ol.

## Revendications

1. Composition de fragrance comprenant du 4,7-diméthyloctan-3-ol.

2. Composition de fragrance selon la revendication 1, dans laquelle la quantité de 4,7-diméthyloctan-3-ol contenue à l'intérieur de celle-ci est de 0,01 à 100 % en masse.

3. Composition de fragrance selon la revendication 1 ou 2, dans laquelle la quantité de 4,7-diméthyloctan-3-ol contenue à l'intérieur de celle-ci est de 0,1 à 15 % en masse.

4. Composition de fragrance selon l'une quelconque des revendications 1 à 3, dans laquelle la quantité de 4,7-diméthyloctan-3-ol contenue à l'intérieur de celle-ci est de 0,3 à 5 % en masse.

5. Composition de fragrance selon l'une quelconque des revendications 1 à 4 comprenant en outre au moins l'un sélectionné dans le groupe constitué d'un alcool autre que le 4,7-diméthyloctan-3-ol, d'un ester, d'un carbonate, d'un aldéhyde, d'une cétone, d'un éther, et d'une lactone.

6. Composition de fragrance selon l'une quelconque des revendications 1 à 5 comprenant en outre une huile.

7. Composition de fragrance selon l'une quelconque des revendications 1 à 6 comprenant en outre un tensioactif.

8. Composition de traitement de tissu comprenant une composition de fragrance selon l'une quelconque des revendications 1 à 7.

9. Adoucissant comprenant une composition de fragrance selon l'une quelconque des revendications 1 à 7.

10. Produit cosmétique comprenant une composition de fragrance selon l'une quelconque des revendications 1 à 7.

11. Composition de nettoyage comprenant une composition de fragrance selon l'une quelconque des revendications 1 à 7.

12. Procédé d'utilisation du 4,7-diméthyloctan-3-ol comme composant de fragrance.

13. Procédé de production du 4,7-diméthyloctan-3-ol, comprenant une étape de mise en oeuvre d'une réaction aldolique croisée en utilisant de l'isovaléraldéhyde et de la 3-pentanone, et ensuite une déshydratation et une réduction pour produire le 4,7-diméthyloctan-3-ol.

14. Le 4,7-diméthyloctan-3-ol.
